(19) Europäisches Patentamt
European Patent Office
Office européen des brevets

(11) **EP 4 141 446 A1**

(12) **EUROPEAN PATENT APPLICATION**
published in accordance with Art. 153(4) EPC

(43) Date of publication:
**01.03.2023 Bulletin 2023/09**

(21) Application number: **21792817.5**

(22) Date of filing: **20.04.2021**

(51) International Patent Classification (IPC):
***G01N 33/543*** (2006.01)    ***G01N 33/569*** (2006.01)

(52) Cooperative Patent Classification (CPC):
**G01N 33/543; G01N 33/569**

(86) International application number:
**PCT/KR2021/004967**

(87) International publication number:
**WO 2021/215803 (28.10.2021 Gazette 2021/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.04.2020 KR 20200048361**

(71) Applicants:
• **Seoul National University R & DB Foundation Seoul 08826 (KR)**
• **Korea Institute of Machinery & Materials Daejeon 34103 (KR)**

(72) Inventors:
• **KIM, Ji Eun**
**Seoul 08826 (KR)**
• **NAM, Jwa Min**
**Seoul 06716 (KR)**
• **KIM, Sungi**
**Seoul 08098 (KR)**
• **AHN, Jun Hyoung**
**Daejeon 35265 (KR)**
• **LEE, Jae-Jong**
**Daejeon 34092 (KR)**

(74) Representative: **Elkington and Fife LLP**
**Prospect House**
**8 Pembroke Road**
**Sevenoaks, Kent TN13 1XR (GB)**

(54) **LIPID NANOPILLAR ARRAY-BASED IMMUNOASSAY**

(57)     Provided are: an array, a kit, and/or a device which are for analyzing, identifying, detecting, and/or visualizing target particles, and/or determining the presence or absence of the target particles, and use a substrate having an uneven surface coated with a lipid bilayer; and a method using same.

【FIG. 3c】

**Description**

[TECHNICAL FIELD]

Cross-reference to related applications

The present application claims the priority based on Korean Patent Application No. 10-2020-0048361 filed on April 21, 2021, and the all the contents disclosed in the document of the corresponding Korean Patent Application are incorporated by reference as a part of the present description.

Provided are an array, a kit, and/or a device for analyzing, identifying, detecting, and/or visualizing target particles, and/or determining the presence or absence of the target particles, using a substrate having an uneven surface coated with a lipid bilayer; and a method using the same.

[BACKGROUND ART]

Since infectious diseases, particularly viral infections, have threatened human health and caused huge economical losses globally, developments of a rapid, sensitive and selective virus detection platform become highly demanded.

Since viruses manipulate and inhibit the host immune system, viral pandemics, such as coronaviruses, African swine fever, Ebola, and influenza, threaten the lives of humans and livestock. Viral pandemics are mainly induced by hosts with undiagnosed infections. To treat the infected individuals in a timely, precise manner and prevent serious epidemics, rapid, sensitive and selective virus detection using a straightforward method is required.

Widely used virus detection methods are based on polymerase chain reaction (PCR) that amplifies target genes, Enzyme-linked immunosorbent assay (ELISA), and rapid influenza diagnostic test (RIDT) that detect whole virus. Although whole virus detection methods are straightforward and do not require further sample processing, they are time-consuming and labor-intensive. ELISA (Enzyme-linked immunosorbent assay) is mainly used for total virus detection because of the convenience and simplicity of the assay protocol. Conventional chip-based biosensors assay for virus detection, such as ELISA, immobilize the capturing molecules (e.g., antibodies) on a substrate for a target detection, and require laborious downstream labelling which transforms the concentration of the captured target into an optical signal. Therefore, ELISA has a limitation that it cannot offer high sensitivity, high specificity, and reliable detection and quantification for viral targets within a short period of time.

Therefore, there is a need to develop a technology capable of detecting and/or quantifying a target particle with high sensitivity more simply and promptly.

[DISCLOSURE]

[TECHNICAL PROBLEM]

The present description provides a lipid nanopillar array-based immunosorbent assay (LNAIA) technology for rapid and sensitive quantitative detection of virus. The three-dimensional structure (3D) nanopillar array and a fluid-fluorophore labeled antibody on the LNAIA platform may facilitate efficient and rapid target binding by inducing fluorescence-antibody clustering on surface of target particle so that the target particle can be detected with very high sensitivity even under a conventional fluorescence microscope.

An embodiment provides an array, a kit, and/or a device for analyzing, identifying, detecting, and/or visualizing a target particle, and/or determining presence or absence of a target particle, comprising:

   a substrate with uneven surface,
   a lipid bilayer coating the uneven surface, and
   at least one (e.g., at least two or plural) capturing substance capable of binding to the target particle, wherein the capturing substance is localized in the lipid bilayer with fluidity.

Another embodiment provides a kit for analyzing, identifying, detecting, and/or visualizing a target particle, and/or determining presence or absence of a target particle, comprising two or more of the arrays.

When the target particle of the array or the kit is a virus, the array or the kit may be applied as an array or kit for diagnosing virus infection or a disease caused by a virus infection.

Another embodiment provides a method of analyzing, identifying, detecting, and/or visualizing a target particle, and/or determining presence or absence of a target particle.

The method may comprise:

contacting a sample with the array or the kit, or supplying a sample to the array or the kit; and

observing, measuring, detecting, identifying, and/or quantifying a signal generated from the array or the kit.

The signal may be generated from the capturing substance labeled with a signaling material, wherein the capturing substance is contained in the array or the kit.

In an embodiment, when the target particle is a virus, the method may be applied as a method of diagnosing virus infection or a disease caused by a virus infection.

[TECHNICAL SOLUTION]

The present description provides a lipid nanopillar array-based immunosorbent assay (LNAIA) technology capable of more rapid and sensitive virus detection and/or quantitative analysis using a substrate having a nanopillar pattern, the surface of which is covered with a supported lipid bilayer containing antibodies which are labeled with fluorescence and contained in the lipid bilayer with fluidity. A method of detecting a target particle provided in the description, which uses fluorescence-labeled antibodies with fluidity and a nanopillar array structure with tertiary structure, may be characterized in that a plurality of labeled antibodies that fluidly move on the surface of the tertiary structure bind to a target particle on multiple sites thereof, to concentrate fluorescence signal around the particle, thereby being capable of a rapid, stable, and efficient analysis of a target particle is possible even with a fluorescence microscope setup of conventional specifications without need to use expensive high-performance equipment. The LNAIA suggested herein can detect and/or quantify a target particle (e.g., a virus) in a shorter time with a high level of sensitivity, which could not be achieved with an existing ELISA platform.

In this description, the term "two or more, or plural" may be used for excluding the case that only one exists, or covering all numerical range of at least two, and for example, may refer to 2 to about $10^{12}$, 2 to about $10^{11}$, 2 to about $10^{10}$, 2 to about $10^9$, 2 to about $10^8$, 2 to about $10^7$, 2 to about $10^6$, 2 to about $10^5$, 2 to about $10^4$, 2 to about $10^3$, 2 to about $10^2$, or 2 to about 10, but not be limited thereto.

An embodiment provides an array, a kit, and/or a device for analyzing, identifying, detecting, and/or visualizing a target particle, and/or determining presence or absence of a target particle, comprising:

a substrate with uneven surface,

a lipid bilayer coating the uneven surface, and

at least one (e.g., at least two or plural) capturing substance capable of binding to the target particle, wherein the capturing substance is localized in the lipid bilayer with fluidity.

The capturing substance may be labeled with a signaling material (e.g., fluorescent material, etc.) generating detectable signal (e.g., fluorescent signal, etc.).

The array may be used for analysis, identification, detection, and/or visualization of two or more target different particles, and/or determination of presence or absence of two or more different target particle, and in this case, the capturing substance may comprise a combination of one or more capturing substances, each of which binds to each target particle of the two or more different target particles.

Another embodiment provides a kit or device for analyzing, identifying, detecting, and/or visualizing a target particle, and/or determining presence or absence of a target particle, comprising the array in the number of at least one or at least two. The at least one or at least two arrays comprised in the kit or device may be for analyzing, identifying, detecting, and/or visualizing a same target particle to each other and/or determining presence or absence of a same target particle to each other, or for analyzing, identifying, detecting, and/or visualizing two or more difference same target particles from each other and/or determining presence or absence of two or more difference target particles from each other. The kit or device may further comprise a means for detecting a signal (fluorescent signal), in addition to the array.

When the target particle of the array or kit is a virus, the array or kit may be applied as an array or kit for diagnosing virus infection or a disease caused by a virus infection.

Another embodiment provides a method of analyzing, identifying, detecting, and/or visualizing a target particle, and/or determining presence or absence of a target particle.

The method may comprise:

contacting a sample with the array or kit, or supplying a sample to the array or kit; and

observing, measuring, detecting, identifying, and/or quantifying a signal generated from the array or kit.

The signal may be generated from the capturing substance labeled with a signaling material, wherein the capturing substance is contained in the array or kit.

In an embodiment, when the target particle is a virus, the method may be applied as a method of diagnosing virus infection or a disease caused by a virus infection.

In the array, kit, and/or method using the same, when a sample to be analyzed is contacted (applied) to the array or kit, plural signaling material-labeled capturing substances (for example, fluorescence-labeled antibodies) located in the lipid bilayer coating the uneven surface of the substrate are distributed on the surface with fluidity in three-dimensions. The plural capturing substances (antibodies) bind to one target particle (e.g., one virus particle) through a three-dimensional interaction (e.g., antigen-antibody interaction), and are surrounding and concentrated on the surface of the target particle, to generate strong (concentrated) signal, whereby it is possible to detect (identify) the target particle in the sample even with naked eye or a conventional equipment (for example, a conventional fluorescence microscope) setup of conventional specifications without need to use expensive high-performance equipment.

Hereinafter, the present invention will be described in more detail:

As used herein, the term "array" may refer to a structure having a predetermined pattern by a four-way (up, down, left, right) repeating arrangement of a plurality of three-dimensional structures, and may be interchangeable with similar terms such as chips.

The substrate may be a solid substrate, wherein the surface thereof may be hydrophilic or modified to have hydrophilicity, to enable a lipid bilayer coating on the surface. In an embodiment, the substrate may be of a material selected from the group consisting of glass, silica, silicon (e.g., a silicon wafer), mica, and all solid substrates with surface modified with a self-assembled monolayer (SAM) of a hydrophilic molecule (e.g., having a functional group, such as, -OH, -COOH, and the like, at its terminus), and may be optionally modified so as to have hydrophilicity, but not be limited thereto.

The uneven surface refers to a surface that is not flat, and may refer to a surface on which a three-dimensional structure including protrusions and/or depressions is formed (or irregularities are formed).

In an embodiment, the uneven surface may be formed by one or more (e.g., two or more, or a plurality of) three-dimensional structures (protrusions, depressions, or a combination thereof). In an embodiment, the uneven surface may comprise one or more (e.g., two or more, or a plurality of) pillars, one or more (e.g., two or more, or a plurality) grooves, or a combination thereof. The size (diameter, width, height, depth, etc.) of the three-dimensional structure, the interval between adjacent three-dimensional structures, and the like may be determined according to the size of the target particle to be detected.

In an embodiment, in order to facilitate three-dimensional interaction between one or more (e.g., two or more or plural) capture substances on a substrate having a surface that is uneven and coated with lipid bilayer having fluidity and one or more (e.g., two or more or plural) materials (e.g., protein, etc.) on surface of the target particle, the diameter, height, and/or depth of the three-dimensional structures formed on the uneven surface of the substrate (e.g., the diameter and/or height of the filler, the diameter and/or depth of the grooves) and/or the distance (interval) between the three-dimensional structures may be greater than the size (e.g., diameter) of the target particle. For example, the diameter, height, and/or depth of the three-dimensional structures and/or the distance between the three-dimensional structures may be about 1.2 times or more, about 1.5 times or more, about 1.7 times or more, about 2 times or more, about 2.2 times or more, or about 2.5 times or more, of the average diameter of the target particle, but not be limited thereto (the upper limit may be appropriately selected according to a intended purpose, for example, about 100 times, about 50 times, about 30 times, about 20 times, about 10 times or about 5 times, but not be limited thereto) .

In an embodiment, the substrate with uneven surface may be a solid substrate comprising one or more (e.g., two or more, or plural) nanopillars (protrusions having a diameter of 1-1000 nm and/or a height of 1-1000 nm) on the surface.

The shape of the protrusion (e.g., nanopillar) and/or the depression (groove) may not be particularly limited, and may be polygonal (e.g., hexagonal, pentagonal, tetragonal, trigonal, octagonal, etc.), circular, oval, etc., based on the shape of the horizontal cross-section, and for example, may be hexagonal, but not be limited thereto.

The uneven surface of the substrate is coated with a lipid bilayer. As used herein, the term "lipid bilayer" refers to a membrane constructed with two layers of lipid molecules. The lipid bilayer may have a structure and/or thickness similar to a naturally occurring membrane, for example, a cell membrane, a nuclear membrane, a viral envelope, and the like. For example, the thickness of the lipid bilayer may be 10 nm or less, for example, about 1 nm to about 10 nm, about 2 nm to about 10 nm, about 3 nm to about 10 nm, about 5 nm to about 10 nm, about 7 nm to about 10 nm, about 1 nm to about 8 nm, about 2 nm to about 8 nm, about 3 nm to about 8 nm, about 5 nm to about 8 nm, about 1 nm to about 6 nm, about 2 nm to about 6 nm, about 3 nm to about 6 nm, or 2.5 nm to 3.5 nm, but not be limited thereto. The lipid bilayer may be formed by a lipid molecule having a hydrophilic head and a hydrophobic tail. When the lipid molecules are exposed to aqueous environment, they are self-aligned to form a bilayer in which the hydrophilic head faces outward in contact with the aqueous environment and the hydrophobic tail faces inward (between the two layers). The lipid molecule may have a compound having carbon atoms of C14 to C50. The lipid molecule may be a phospholipid. The phospholipid may have carbon atoms of C16 to C24. The phospholipids may be obtained artificially or from nature. The phospholipid may be a diacylglyceryl phospholipid, for example, phosphatidic acid, phosphatidyl ethanolamine, phosphatidyl choline, phosphatidyl serine, phosphatidyl glycerol, phosphatidyl inositol, or a combination of two or more thereof.

For example, the lipid molecule may be at least one selected from the group consisting of dioleoyl phosphatidy lcholine (1,2-dioleoyl-sn-glycero-3-phosphocholine; DOPC), dioleoyl phosphatidyl ethanolamine (1,2-dioleoyl-sn-glycero-3-phosphoethanolamine; DOPE), dihexadecanoyl phosphatidyl ethanolamine (1,2-dihexadecanoyl-sn-glycero-3-phosphoethanolamine; DHPE), and the like.

In the array, kit, and/or device provided herein, the uneven surface of the substrate is coated with a lipid bilayer, and fluidity of the lipid bilayer gives mobility to two or more (plural) capture substances located inside the lipid bilayer, thereby concentrating the capturing substances around the target particle.

In an embodiment, the target particle may be selected from all particles having on their surfaces one or more (e.g., two or more or plural) materials (e.g., proteins, nucleic acid molecules, etc.) capable of being captured (detected, recognized, and/or bound) by the capture substance. For example, the target particle may be at least one (e.g., two or more, or plural) selected from the group consisting of viruses including influenza viruses (e.g., influenza A virus subtype H1N1, influenza A virus subtype H3N2, avian influenza virus (influenza A virus subtype H5N1 , Viruses including influenza A virus subtype H7N9 , etc. )), coronaviruses (e.g., Middle East Respiratory Syndrome coronavirus (MERS-CoV), Severe Acute Respiratory Syndrome coronavirus (SARS-CoV), SARS-CoV-2, etc.), and the like; bacteria; bacteriophages; cells; protein particles (e.g., aggregates, etc.); nucleic acid molecules (e.g., DNA, RNA, etc.); and the like.

The capture substance may be one or more selected from the group consisting of substances, capable of (specific) binding to a material (e.g., a protein, a nucleic acid molecule, etc.) on the surface of the target particle, such as an antibody, an aptamer, etc. The capture substance may be labeled with an appropriate detectable label (signaling material), such as a fluorescent material, a plasmon metal nanoparticle, and the like. The array/kit provided herein may comprise two or more (plural) capture substances for one kind of target particle. In an embodiment, when the target particle is a virus or a cell, the capture substance may be at least one selected from the group consisting of antibodies, aptamers, and the like, that specifically bind to proteins (e.g., various receptors, antigens, etc.) exposed on the surface of the virus or cell.

The labeled capture substance may be located inside a lipid bilayer coating the uneven surface, and capable of moving (having fluidity) inside the lipid bilayer along the surface. Two or more labeled capture substances can move to an area of the target particle where is located in the three-dimensional space formed by bottom of the uneven surface and sidewall of a three-dimensional structure (protrusion; e.g., nanopillar), surround the target particle, and bind to a plurality of capturable materials on the surface of the target particle, thereby being concentrated around the target particle and generating a strong signal. For this reason, it is possible to detect and/or visualize the target particle even with the naked eye or conventional detection equipment (e.g., a fluorescence microscope) without expensive and high-performance equipment.

In the kit and/or device provided herein, a signal (fluorescent signal) detection means, that may be optionally included in the kit/device, in addition to the array, may be any detection means available for detecting the signal, and may be a microscope (e.g., a fluorescence microscope, etc.) of conventional level (e.g., 40 ~400x or 40-100x magnification), but not be limited thereto. This is to explain the excellent visualization effect and detection sensitivity of the detection technology provided in this specification due to the high signal intensity by a three-dimensional concentration of the signal, and should not be interpreted as meaning to exclude the use of high-performance detection means for signal detection.

In the method provided herein, the sample may be a biological sample obtained or isolated from a subject and/or an environmental sample (water, soil, air, etc.). The biological sample may be at least one selected from the group consisting of cells, blood, lymph, saliva, sputum, snivel, urine, feces, and other body fluids, but not be limited thereto. The subject may be selected from mammals, such as humans, livestock (e.g., cows, pigs, horses, sheep, goats, dogs, cats, etc.), birds (e.g., chickens, ducks, geese, turkeys, ostriches, quails, etc.), but not be limited thereto.

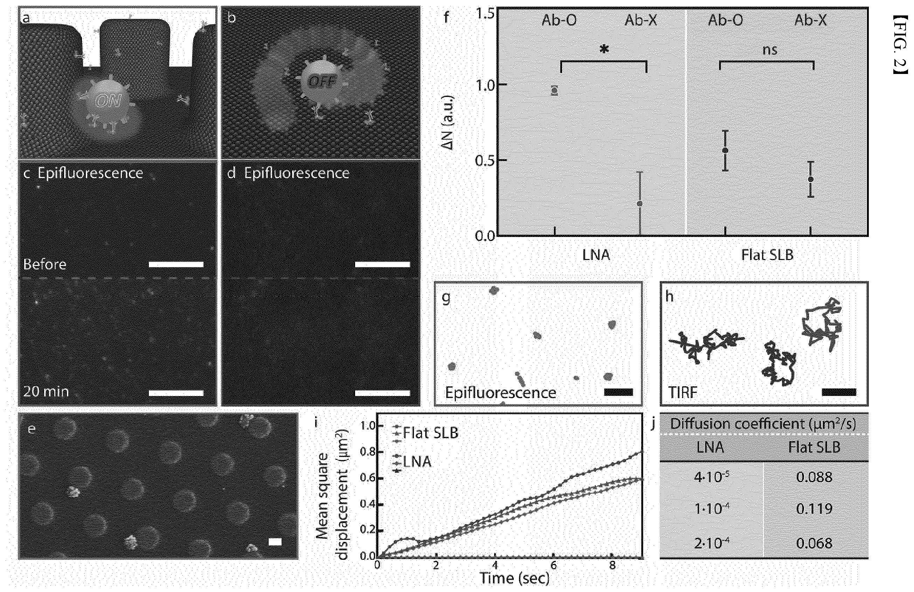

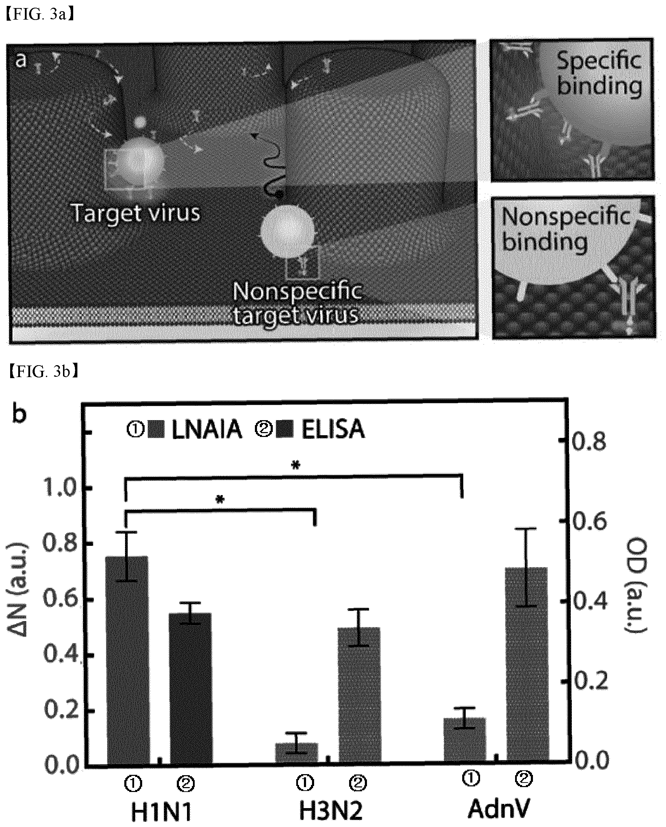

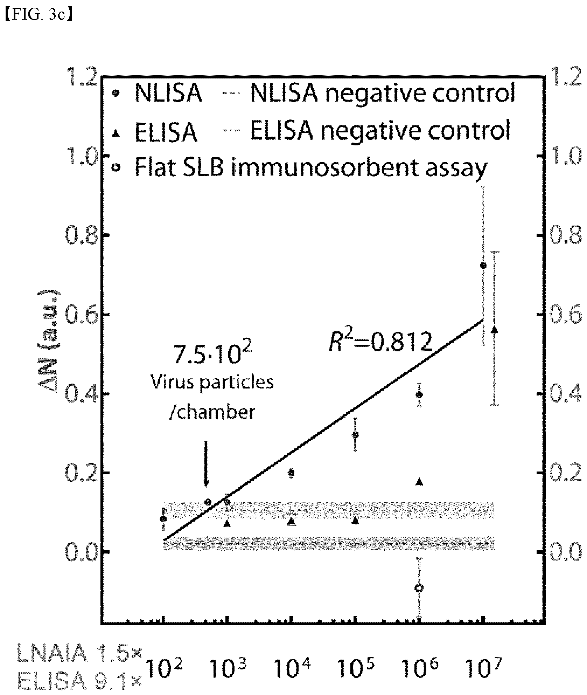

The detection sensitivity when detecting a target particle by the kit, device, and/or method provided herein may be about 2 times or higher, about 5 times or higher, about 10 times or higher, about 20 times or higher, about 30 times or higher, about 40 times or higher, or about 50 times or higher (e.g., about 100 times or higher, about 1,000 times or higher, about 5,000 times or higher, or about 10,000 times or higher), compared to that of the case of using a flat substrate and/or compared to existing similar measurement method (e.g., ELISA, etc.) (see FIGS. 2, 3b, 3c, etc.). In a specific example, when the target particle is a virus, the detection limit of detection using the lipid bilayer coated nanopillar substrate provided in the present description is about 150 virus particles, which is about 40 times higher than that of the conventional ELISA.

The detection time required for detecting a target particle using the kits, devices, and/or methods provided herein may be less than about 3 hours, for example, about 5 minutes to about 300 minutes, about 5 minutes to about 270 minutes, about 5 minutes to about 240 minutes, about 5 minutes to about 210 minutes, about 5 minutes to about 180 minutes, about 5 minutes to about 150 minutes, about 5 minutes to about 120 minutes, about 5 minutes to about 90 minutes, about 5 minutes to about 60 minutes, about 5 minutes to about 50 minutes, about 5 minutes to about 40 minutes, about 5 minutes to about 30 minutes, or about 25 minutes, which is significantly shorter than that of a conventional similar measurement method (e.g., ELISA).

In addition, since the kit, device, and/or method provided herein generate a target particle-specific detection signal using a plurality of labeled capture substances introduced into the lipid bilayer coated on the substrate surface, it is not required to use additional dye (fluorescent dye, etc.). Due to these characteristics, the kit, device, and/or method provided herein has advantage that an accurate analysis can be achieved without a separate washing step of unreacted dye, which is essential step of a conventional method using a dye. Considering that a washing step to remove an unreacted target (cells, etc.), dye, and the like is required in conventional detection using a dye, such characteristics may be advantageous to shorten the time and/or effort required for the analysis and increase the analysis accuracy.

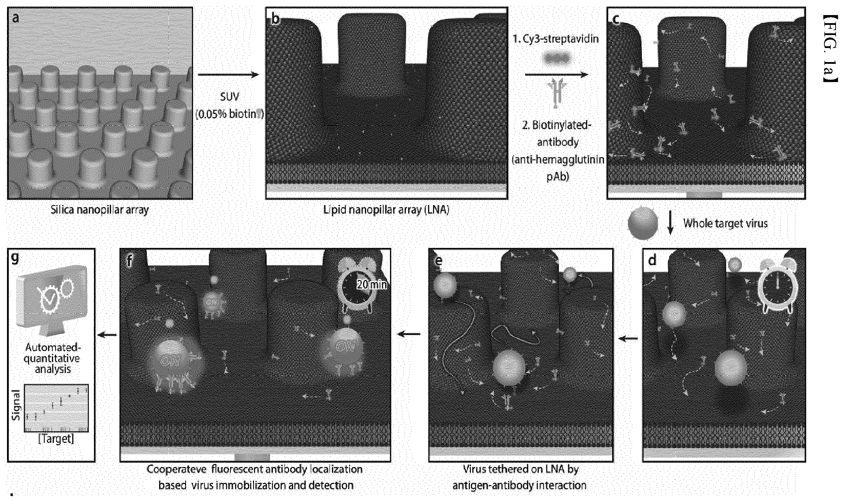

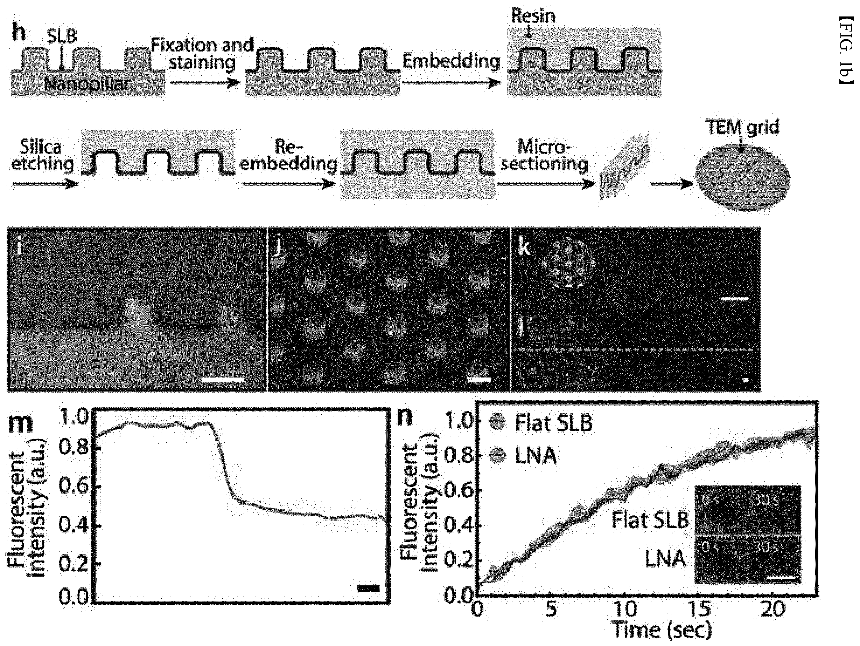

A use for virus detection of the lipid nanopillar array-based immunosorbent assay (LNAIA) provided in the present description may be illustrated as a representative example with reference to FIGS. 1A and 1B, as follows: Supported lipid bilayer (SLB), synthetic fluid lipid bilayer on a solid substrate, may be hybridized with nanostructures and utilized for various biotechnological applications because of the dynamic movement of the lipids similar to cell membrane. Moreover, plasmonic nanoparticles can be modified to the SLB for imaging and analyzing the interactions between nanoparticles in a single-particle level. The present description provides an immunosorbent whole virus assay on a nanofabricated SLB, termed as a lipid nanopillar array-based immunosorbent assay (LNAIA), inspired by the dynamic movement and clustring of receptors on a host cell membrane when virus is engulfed into the cell through endocytosis (FIGS. 1a and 1b). The biotinylated antibodies with Cy3 fluorecent dyes (Cy3-antibodies) tetherd onto a lipid nanopillar array (LNA) by streptavin linkers can dynamically move along on the LNA (FIG. 1a (b-c)). In LNAIA, when target viruses are introduced to the LNA, they are captured by the freely diffusing Cy3-antibodies and eventually surrounded by multiple Cy3-antibodies on the bottom surface and the side wall of the pillars that generate three-dimensional polyvalent interactions with the antibodies (FIG. 1a (d-f)). Such three-dimensional polyvalent interactions are capable of creating localized and immobilized fluorescent spots that can be clearly distinguishable from the fluorescent background with a routinely used epifluorescence microscope. Because the number of hemgglutinin (HA) molecules on the viral surface is greater than neuraminidase (NA) by an order of magnitude, a strong fluorescent signal can be obtained by the efficient localization of multiple HA-specific, Cy3-antibodies.

The nanopillar array underlying SLB may be engineered to form nanostructural protrusions, enhancing rapidity and sensitivity of the target virus detection by providing a larger surface area and the 3D labyrinth-like environment with dynamic movement of fluorophescent antibodies that facilitate the target viruses to abundantly collide with the tethered Cy3-labelled antibodies. The silica nanopillar array fabricated by resist-free direct UV/thermal nanoimprint lithograpy provides sufficient hydrophilicity for stably forming SLB on the substrate. The nanopillar array is 200 nm in diameter and height and have 200 nm gaps between pillars, creating a hexagonal array (FIGS. 1a (a), 7, and 8), so that maximizes the collisions between viruses and the nanopillars and enables the fast, efficient capture of an 80-120 nm sized influenza virus with multiple antibodies by three-dimensional polyvalent antigen-antibody interactions.

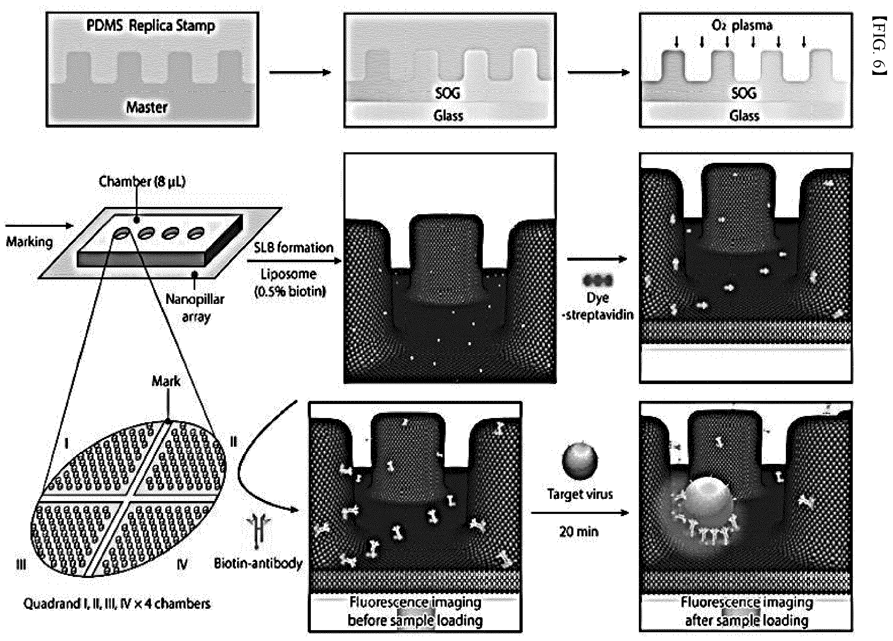

In a typical experiment, LNAIA requires ~25-min assay time after loading analytes, and the stepwise assay procedure is llstrated in FIGS. 1a, 1b, and 6. In the attached 8-$\mu$L silicon chamber on a hydrophilic silica nanopillar array, small unilamellar vesicles (SUV) with 0.5%(w/v) biotinylated-DOPE in 150 mM PBS was introduced. The ruptured vesicles in the chamber then form lipid bilayer on a supporting nanopillar array. After washing excess SUVs, Cy3-labled streptavitidin and biotinylated polyclonal HA specific antibody were introduced to the chamber. Before loading analytes, we imaged the substrate with a conventional fluorescence microscope. 20 min after loading 8 $\mu$L of analytes, we imaged and quantified the number of newly formed fluoresent spots in the chamber with an automated imageJ software.

In summary, the present description provides a new type of immunosorbent assay that can quickly (~25 min) and reliably detect viral targets at very low concentrations (as low as about 150 viruses) with an excellent dynamic range (at least 5 orders of magnitude in concentration) based the fluorescent antibody-modified LNA with a routinely used fluorescence microscope. It is demonstrated that the engineering of nanopillar structures with dimensions comparable to the virus could greatly enhance the performance of dynamic and cooperative three-dimensional interactions between viral target and fluidic antibody. Lipid bilayer-coated nanopillar substrate can boost the binding kinetics and specificity in virus sensing over the typical physical molecular binding constants of antibodies with conventional assay platforms (e.g., ELISA). Only localized and concentrated fluorescence signals, generated from specific multivalent bindings between virus and antibodies, can be reliably detected with a conventional epifluorescence microscope at extended exposure time of 300ms - this can efficiently eliminate false positive signals by averaging background signal and selectively collect true positive signals. A multiplexed detection of target virus in LNAIA using multiple fluorescent channels could resolve many of the difficulties that arise from cross-reactive antibodies in other multiplex immunoassays. It is possible to significantly shorten the detection time and minimize assay steps by adopting a 3D-structured labyrinth substrate that maximizes collisions between targets and capture molecules and integrating the transducer and the detector of a biosensor into a single unit and adapting an automated signal analysis. The shortened detection time and minimal assay process on a widely used detection setup can facilitate the use of this assay for quick, straightforward diagnostic test with high sensitivity.

[EFFECTS OF THE INVENTION]

The LNAIA of the present description provides a new detection strategy and platform for fast, sensitive, selective, and reliable detection of targets with minimal sampling handling. In addition, the LNAIA has high sensitivity and wide dynamic range. Therefore, the LNAIA can be readily applied to various actual uses including development of rapid-reaction kits for various and different targets such as proteins, DNA, and the like.

[BRIEF DESCRIPTION OF THE DRAWINGS]

FIG. 1a is a schematic view showing a process of a nanopillar-supported lipid bilayer immunosorbent assay (LNAIA) according to an embodiment of the present description and characteristics of the nanopillar-supported lipid bilayer substrate.

In FIG. 1b, h) schematically shows an experimental procedure of transmission electron microscopy (120 kV, Talos L120C, FEI, Hillsboro, United States) of the lipid bilayer formed on the nanopillar-patterned substrate, i) shows a TEM image of the micro-sectioned nanopillars, j) shows a SEM image of a tilted nanopillar substrate, k) shows SEM image of the pillar-to-flat boundary area, 1) shows a fluorescence microscopic image of 1% Texas Red-modified SLB formed on the pillar-to-flat boundary area, m) shows a fluorescence intensity line profile on the dotted line shown in FIG. 1a (e), and n) shows a fluorescence recovery after photobleaching (FRAP) results of the nanopillar patterned substrate and a flat substrate (diffusion coefficients of flat SLB and nanopillar SLB are -0.34 $\mu$m2/s and -0.35 $\mu$m2/s, respectively. The colored areas in the plots represent mean standard errors. The scale bars in i-j, and k-n are 200 nm and 5 $\mu$m, respectively).

FIG. 2 shows virus capture and mobility results on nanopillar and flat SLB substrate, wherein a) illustrating a trapped virus on the nanopillar SLB after assay, b) schematically shows a freely moving virus on a flat SLB after assay, c) is an epifluorescence microscopy images on lipid nanopillar array (LNA) before and after assay, d) is an epifluorescence microscopy images on flat SLB before and after assay, e) is a SEM image of substrate after assay, wherein the SEM image was acquired after freeze-drying, f) shows LNAIA results on each SLB with antibody (Ab-O) and without antibody (Ab-X) (the p-value between Ab-O and AB-X on the LNA was 0.0286. *p < 0.05 (one-tailed Mann-Whitney U test)), g) shows trajectory of fluorescence spots on the nanopillar SLB via an epifluorescence microscope, h) shows trajectory of fluorescence spots on flat SLB via TIRF microscope, i) is a graph showing mean square diameter of fluorescence spots on each SLB after assay, and j) shows diffusion coefficient.

FIGS. 3a-c show LNAIA detection results for H1N1 virus as a target particle, wherein, 3a schematically shows an elimination of false positive results by weak fluorescence signal-based paucivalent nonspecific bindings, 3b is a graph showing LNAIA and ELISA results for H1N1 target and non-target viruses ($1.5*10^5$ particles/chamber for LNAIA and $9.1*10^5$ particles/chamber for ELISA) (in LNAIA, the p-value for H1N1 compared to H3N2 and AdnV was 0.028. *$p$ < 0.05 (one-tailed Mann-Whitney U test)), and 3c is a graph results of quantitative analysis of H1N1 virus detection in human serum using LNAIA and ELISA (after virus loading, ELISA required roughly 6 hours while LNAIA required 25 minutes, including data analysis. Negative control was conducted with $1.5 \cdot 10^5$ particles/chamber and $9.1 \cdot 10^5$ particles/chamber of adeno virus for LNAIA and ELISA respectively, and the plotted data represent mean $\pm$ standard deviation).

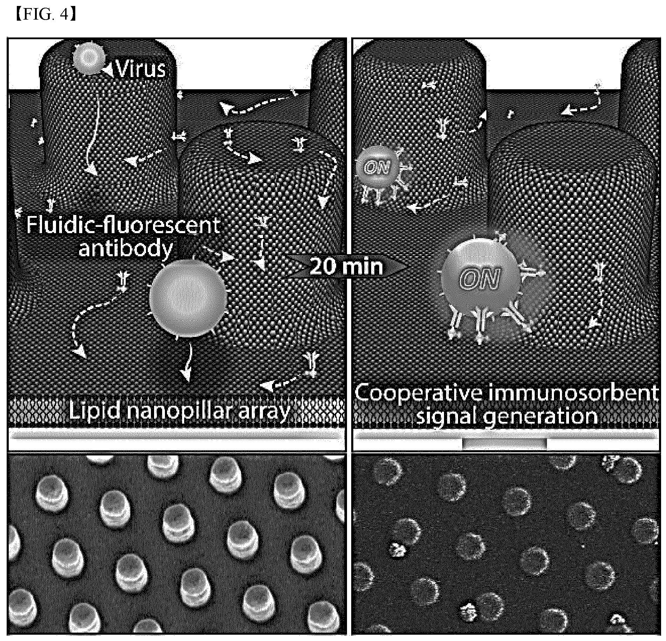

FIG. 4 shows a schematic diagram of a virus detection process using LNAIA (upper part), and an SEM image of the nanopillar substrate corresponding to the upper schematic diagram (lower part).

FIG. 5 shows comparison between the procedures of LNAIA and ELISA.

FIG. 6 shows a stepwise depiction of a LNAIA setup according to an embodiment of the present description.

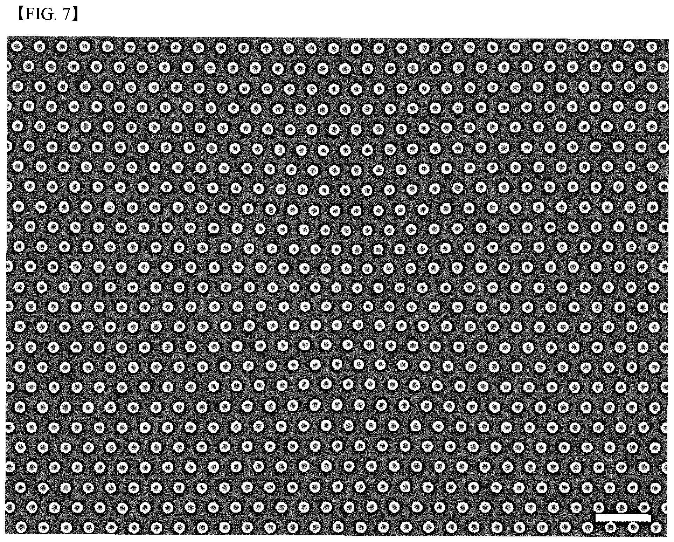

FIG. 7 is a large-scale SEM image of a nanopillar pattern (Scale bar is 1 $\mu$m).

FIG. 8 is a tilted SEM image of a nanopillar substrate (Scale bar is 200 nm).

FIG. 9 is a TEM images of sectioned LNA (Scale bars are 1 $\mu$m).

[MODE FOR INVENTION]

Hereinafter, the present invention will be described in more detail by way of examples.

However, the following examples are only illustrative of the present invention, and the scope of the present invention should not be limited thereto.

Example 1. Preparation of LNAIA kit

1.1. Preparation of small unilamellar vesicles (SUVs)

In a 50 mL round-bottomed flask, 97.45 mol% DOPC(1,2-Dioleoyl-sn-glycero-3-phosphocholine), 0.05 mol% biotinylated DOPE(1,2-Dioleoyl-sn-glycero-3-phosphoethanolamine), and 2.5 mol% PEG-DOPE (Molecular Probes, USA) were mixed in chloroform. The prepared lipid solution was evaporated with a rotary evaporator. For the FRAP experiment (Fluorescence recovery after photo bleaching) and fluorescence imaging of SLB (Supported lipid bilayer) in the pillar-to-flat boundary area, 1 mol% FITC-DHPE (1,2-dihexadecanoyl-sn-glycero-3-phosphoethanolamine) and 1 mol% TexasRed-DHPE were included in the round-bottomed flask, respectively. Using a stream of N2 gas, the lipid film was completely dried. After being thoroughly dried, the solution was resuspended in DI water (Nanopure water with a minimum resistance >18 M$\Omega$ cm$^{-1}$), transferred to a cryo-tube, and subjected to three freeze-thaw cycles. The final lipid concentration was 4 mg/mL. The lipid solution was extruded 21 times through a polycarbonate (PC) membrane (Whatman, Fisher Scientific) with a pore diameter of 100 nm at 25°C. Liposomes of approximately 100 nm were obtained and kept at 4°C until use.

1.2. Antibody biotinylation.

0.2 mg/mL H1N1 antibody (Influenza A H1N1 (A/Puerto Rico/8/1934) Hemagglutinin, Sinobiological) in 150 mM PBS (50 $\mu$L) and 20 $\mu$M NHS-biotin (N-hydrocysuccinimidobiotin Thermo Scientific, USA) in DMSO (5 $\mu$L) were mixed and incubated for 2 hours at room temperature. Non-reacted biotin was removed with desalting columns (Zeba Spin desalting columns 7K MWCO, Thermo Scientific, Rockford, IL, Rockford). The prepared reacting product described above was loaded in washed desalting columns and 150 mM PBS was added as a stacker. The column was centrifuged at 1500 g for 2 minutes in eppendorf tube, and the biotin-modified antibody in the solution collected bottom of the tube was bound to streptavidin (STV) on the lipid bilayer.

**1.3. Nanopillar pattern fabrication using spin-on-glass and nanoimprint lithography**

A 4-inch glass wafer was cleaned with plasma asher for 10 min. The glass substrate was spin-coated with IC3-200 spin-on-glass (SOG, Futurrex Inc. USA) at 3000 rpm for 30 sec. The hole-patterned polydimethylsiloxane (PDMS; SYLGARD 184, Dow Corning, USA) stamp was 200 nm in diameter, 300 nm in depth, and 400 nm in pitch, and was pressed onto the SOG-coated glass wafer with 2000 kgf cm$^{-2}$ at 150°C for 10 minutes using ANT-6HO2 UV/thermal nanoimprint lithography (KIMM, Korea). Afterwards, the hole stamp was detached from the glass wafer, and a substrate with nanopillars formed on its surface (hereinafter, called as 'nanopillar substrate, nanopillar patterned substrate, or nanopillar array') was obtained (FIG. 1a (a)).

**1.4. Fabrication of LNAIA kit**

To fabricate a virus assay kit, silica nanopillar-patterned substrate was given hydrophilicity by treating the silica nanopillar-patterned substrate prepared in Example 1.3 with plasma asher after washing with ethanol and acetone for 10 times respectively. On the substrate, a sticker chamber (8 $\mu$L) was attached and small unilamellar vesicles (SUVs) prepared in Example 1.1 were mixed with 150 mM phosphate buffered saline (PBS) at the ratio of 1: 1 (v/v) and introduced to the chamber for 30 minutes. After marking a cross line on the bottom of each chamber with a disposable needle and removing excess SUV with 150 mM PBS, 8 $\mu$L of 20 $\mu$M bovine albumin serum (BSA, Sigma Aldrich) in 150 mM PBS was introduced to inactivate the non-covered area on the nanopillar-patterned substrate. After removing excess BSA with 3 washes with 150 mM PBS, 8 $\mu$L of 20 nM Cy3-modified streptavidin (Cy3-STV, Molecular Probes, USA) in 150 mM PBS was introduced for 30 minutes, followed by another wash with 150 mM PBS. Subsequently, biotinylated antibody (Example 1.2) with an O.D. of 0.02 at 280 nm was introduced for 30 minutes. The chamber was then washed three times with 150 mM PBS prior to the assay.

**Example 2. LNAIA**

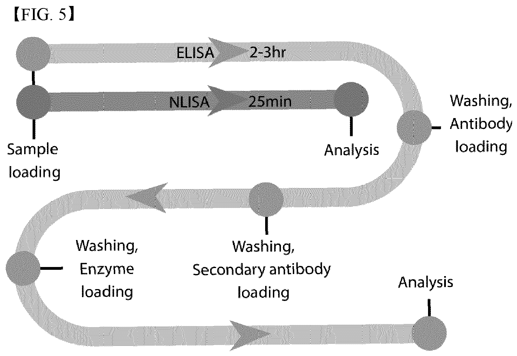

A LNAIA was performed using the LNAIA kit (target virus: H1N1) prepared in Example 1.4. A step wise experimental procedure and LNAIA chamber design is illustratively described in FIGS. 5 and 6. Epifluorescence images of 4 quadrants from each chamber were acquired before and 20 minutes after introducing 8 $\mu$L of $1.8*10^4$ to $1.8*10^9$ viral particles/mL virus (Influenza A/Puerto Rico/8/1934 H1N1 for positive target, and Adenovirus type 5 for negative target) sample with 1 % (v/v) human serum (from human male AB plasma, USA origin, sterile-filtered, Sigma-Aldrich) in PBS to the LNAIA chamber under exposure to a 488 nm laser and 60x lens via TE-2000 (Nikon, Tokyo, Japan). Three images

with an $80 \times 80$ $\mu m^2$ ($512 \times 512$ pixel$^2$) field of view and 100 ms exposure time were stacked. The fluorescence images were analyzed to count $\Delta N$ (the increased number of fluorescent spots in the four quadrants) using the MOSAIC plugin for Image J software. The four $\Delta Nq$ (the increased number of fluorescence spots in each quadrant) from a single well were summated as $\Delta N$.

**Reference Example 1. ELISA (comparative example)**

50 · L of 1.8*10$^4$ to 1.8*10$^9$ viral particles/mL virus (Influenza A/Puerto Rico/8/1934 H1N1 for positive target, and Adenovirus type 5 for negative target) samples with 1 % human serum in coating buffer (0.2 M $Na_2CO_3$/$NaHCO_3$, pH 9.6) were added to individual wells and incubated for 2 hours at room temperature. The wells were washed with 200 · L of PBST (137 mM NaCl, 2.7 mM KCl, 10 mM $Na_2HPO_4$, 0.1 w/v % Tween 20) 3 times and patted dry on a hand towel. The remaining surface of each well was blocked with 200 · L of 1 % BSA in PBS (137 mM NaCl, 2.7 mMKCl, 10 mM $Na_2HPO_4$, 1.8 mM $KH_2PO_4$) for 2 hours at room temperature. Excess BSA was removed with two washes of 200 · L of PBST. 2 · g/mL of primary antibody in PBS with 1% BSA was introduced to each well in a total volume of 100 · L for 2 hours at room temperature. The excess antibody was removed with 4 washes with 200 · L of PBST. 100 · L of secondary antibody with 1 % BSA in PBS was added and incubated for 2 hours at room temperature. The wells were washed 4 times with 200 · L PBST and 50 · L of 3,3',5,5'-tetramethylbenzidine (TMB) was added. After 15 minutes, each well was treated with 50 · L of 2 M of $H_2SO_4$ and analyzed by a microplate reader (Multiple Plate Reader, Victor 3, Perkin Elmer, USA).

**Reference Example 2. Transmission Electron Microscopy (TEM) Assay**

For imaging SLB structure on a nanopillar pattern, the nanopillar labyrinth SLB was fixed in 2% glutaraldehyde and 4% paraformaldehyde in 0.1 M sodium cacodylate buffer (SCB) (pH 7.3) at room temperature for 30 minutes, followed by fixation overnight. The following steps were carried out at 4°C until graded ethanol series. The sample was washed in 0.1 M SCB with 0.2 M sucrose 3 times and post-fixed with reduced osmium (1% osmium tetroxide with 0.8 % potassium ferricyanide in 0.1 M SCB). After washing with distilled water 3 times, en-bloc staining was carried out with 2 % uranyl acetate. After washing the sample with distilled water 3 times, it was dehydrated with 30 %, 50 %, 70 %, 80 %, and 100 % ethanol in series. Next, the ethanol was exchanged for acetonitrile. Sample infiltration was carried out with a 1:1 mixture of acetonitrile and Spurrs' resin for 2 hours, a 1:2 mixture of acetonitrile and resin for 2 hours, and pure resin overnight at room temperature. The resin was exchanged with fresh resin and baked at 70 °C overnight. The bottom silica nanopillar pattern was etched away with 49 % hydrofluoric acid (Sigma Aldrich) in a fume hood, thoroughly washed with distilled water, and dried at 70 °C for 30 minutes. The sample was re-embedded with pure resin and baked at 70 °C overnight. Subsequently, the resin block was sectioned by ultramicrotome (EMUC7, Leica, Wetzlar, Germany) into 70 nm-thick sections, then collected onto a TEM copper grid (Veco Center Reference Square Grid, 200-mesh, Cu, Tedpella, USA) and post-stained with 2% uranyl acetate. The prepared grids were imaged with 120 kV transmission electron microscope (Talos L120C, FEI, Hillsboro, United States).

**Reference Example 3. Scanning Electron Microscope (SEM) Assay**

To determine whether virus detected by LNAIA was attached between the wall and bottom of the nanopillar labyrinth, the substrate was freeze dried post-assay, followed by platinum deposition and imaging by SEM (JSM-7800F Prime, JEOL Ltd, Akishima, Japan).

**Example 3. Confirmation of formation of supported lipid bilayer (SLB) on surface of nanopillar array**

The substrate generated in Example 1.4 by applying the small unilamellar vesicles (SUVs) (Example 1.1) onto the nanopillar-patterned substrate (Example 1.3) was observed via transmission electron microscopy (TEM) (120 kV, Talos L120C, FEI, Hillsboro, United States) and fluorescent microscopy (TE-2000, Nikon, Tokyo, Japan), and the obtained results are shown in FIG. 1b (h-n). As a result, the formation of SLB on the nanopillar array was observed.

SLB was designed to fully cover the side wall and top part of the nanopillars and the bottom of the substrate for efficient virus entrapment between the pillar wall and the bottom surface.

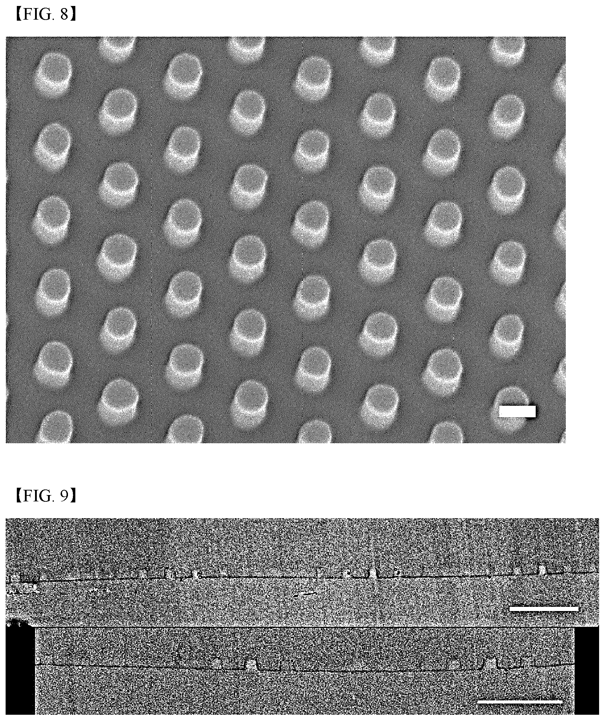

For characterization of the SLB coverage on a nanopillar substrate, the phosphate head groups of SLB were dyed by uranyl acetate, embedded in Spurr's resin, microsectioned via ultramicrotome, and observed via TEM (FIG. 1b (h) and (i), FIGS. 2 and 9). The dark line in FIG. 2 (i) reveals that the dyed supported lipid bilayer closely follows the nanopillar substrate surface.

Due to the high surface area of the nanopatterned array as compared to a flat substrate, the fluorescence intensity of the nanopatterned substrate was much stronger than is seen on a flat surface. The theoretical surface area ratio between the pillar-patterned and flat areas of the LNAIA kit prepared in Example 1.4 was calculated using following Formula 1, for comparison with the fluorescence intensity ratio. The obtained results are shown in FIG. 1b (k-m).

$$\frac{A_{cell}}{A} = 1 + \frac{2hp}{R} = 2.05$$

(Formula 1)

$A_{cell}$ is the total surface area of a unit cell containing a single cylindrical pillar, A is the surface area of a unit cell without a pillar, h is the height of a pillar (200 nm), p is the surface coverage of the pillars, and R is the radius of a pillar (100 nm).

This calculation yielded a ratio of 2.05, which closely matched the measured fluorescence ratio of 1.93, further indicating SLB formation along the nanopillar pattern (FIG. 1b (k-m)).

**Example 4. Virus detection by LNAIA**

A fluorescence recovery (https://doi.org/10.1007/978-l-61779-207-6_26) was conducted after photobleaching (FRAP) experiments to investigate the fluidity of the SLB on a nanopillar substrate, and the obtained results are shown in FIG. 1n (n). A flat SLB kit prepared referring to Example 1.4 using a 4-inch glass wafer substrate without nanopillar array was used for comparison.

The degree of fluorescence recovery after bleaching indicates that SLB covers the nanopillar substrate without losing fluidity. In previous studies combining nanopatterned substrates and SLB, SLB either did not fully cover the nanopattern or formed a suspended structure due to either hydrophobic surface or unsuitable liposome size. In LNAIA provided in the present description, the nanopillar substrate was made of silica to achieve high hydrophilicity and proper dimensions, enabling homogeneous formation of a lipid bilayer along the pattern on the substrate (FIG. 2 (a) and (b)).

Then, localization events of fluidic, fluorescent antibodies around the target virus were monitored via conventional epifluorescence microscopy. During the LNAIA assay discribed in Example 2, fluorescent images before and after a 20-minute sample loading were captured and shown in FIG. 2 (c) and (d). As an H1N1 virion approaches the antibody-modified LNA, multiple polyclonal antibodies bind to the virus, localizing the fluorescent antibodies to an area roughly the size of a single virus. The concentrated antibodies generate a readily-detectable fluorescent spot corresponding to a single virus with a high signal to background noise ratio.

For quantitative virus detection, the increased number of fluorescence spots (ΔN) was automatically counted using a particle-counting software (MosaicSuite particle tracker plugin of image J). It was found that the nanopillar array structure plays an important role in increasing assay sensitivity and target capturing efficiency. The fast, strong colocalization of Cy3-antibody and immobilization of target virus resulted from the three-dimensional antigen-antibody interactions generate a high fluorescence signal to background signal ratio that enables efficient target detection by conventional fluorescence microscopy (FIG. 2 (a) and (c)). On the other hand, the inefficient colocalization of Cy3-antibodies and high lateral mobility of viruses on flat SLB hamper visualization of single virus over a background, and there was nearly no difference in fluorescence signal before and after sample loading (FIG. 2 (b) and (d)). Since the fluorescence-antibody on the substrate on which flat SLB is formed did not aggregate, the fluorescence spot cannot be observed with a conventional fluorescence microscope, and a high-end total internal fluorescence microscope (TIRFm, TE-2000, Nikon, Tokyo, Japan) is required for detecting single-molecule fluorescence-antibody signals.

In addition, after performing the LNAIA assaay of Example 2, the substrate was freeze-dried, and the freeze-dried substrate was observed with SEM (Reference Example 3). The obtained image is shown in FIG. 2 (e). as shown in FIG. 2 (e), the position of virus on LNA is typically between the sidewall of a pillar and the bottom surface, further showing the importance of protruding nanopillar structures with a hexagonal array for efficient capture of viruses.

The LNAIA was performed using SLB with antibody (Ab-O) and SLB without antibody (Ab-X), and ΔN value obtained thereby was shown in FIG. 2 (f). As shown in FIG. 2 (f), in case of LNA (lipid nanopillar array), the ΔN value obtained in the test group with antibody was significantly larger than that of a control group without antibody (p-value of 0.0286), while in case of flat SLB, there is no significant differnece between ΔN values of the test group with antibdy and the control group without antibody (p-value of 0.028).

Trajectories of fluorescence spots on the nanopillar SLB observed via epifluorescence microscope were shown in FIG. 2 (g), and trajectories of fluorescence spots on flat SLB observed via TIRF microscope (TE-2000, Nikon, Tokyo, Japan) were shown in FIG. 2 (h). The trajectories of fluorescence spots shown in FIG. 2 (g) and (h) indicate near-immobilization (LNA; FIG. 2 (g)) and free diffusion (flat SLB; FIG. 2 (h)) of virus particles for LNA and flat SLB surfaces, respectively. While the trajectories of localized fluorescence spots on LNA were observable via conventional epifluorescence microscopy, the trajectories of fluorescence spots on the flat SLB could not be detected via conventional epifluorescence microscopy and were only observable via TIRF microscopy (FIG. 2 (g-j)). The LNA boosts the binding kinetics of the virus to the surface antibodies due to the enlarged 3D surface area and multiple protrusions enhancing the collisions between surface and virus.

**Example 4. Comparison of LNAIA and ELISA**

The results obtaied by performing LNAIA and ELISA referring to Example 2 and Reference Example 2, respectively, were shown in FIGS. 3b and 3c. In case of ELISA, nonspecific viruses, H3N2 and adenovirus (AdnV), were not differentiated from target virus(H1N1) at low concentration (at about $10^5$ particels/chamber or lower) (FIG. 3c). Although the same antibody was used for both LNAIA and ELISA, LNAIA showed significantly higher selectivity compared to ELISA (FIG. 3b).

Such high selectivity of LNAIA is due to the following hallmarks of LNAIA: Signal generation is accomplished by the cooperative interaction between the Cy3-antibody and the viral target. False positive signals (nonspecific bindings) are efficiently eliminated with LNAIA because true positive signals (specific bindings) are only produced when the concentration of Cy3-antibody exceeds the threshold of the fluorescence microscope, and each individual clustered spots can be reliably imaged and analyzed only when virus targets were multivalently captured by fluorescent antibodies (FIG. 3a). In addition, extracellular molecules in analytes cannot be bound by SLB, an artificial cell membrane without membrane proteins (indicating that SLB is an excellent surface with minimal nonspecific bindings). The above results can be further supported by lower background signals with the LNAIA negative control than with the ELISA-based negative control (FIG. 3c).

Importantly, the mean of dN($\Delta$N) values at $10^5$ section with LNAIA display a linear relationship. The detection limit of LNAIA is about 150 virus particles, which showed about 10,000-fold higher sensitivity than commercially available H1N1 ELISA. Moreover, LNAIA requires only 25 minutes while ELISA required 3 hours or more due to laborious antigen binding, labeling and washing procedures (FIG. 3c).

The examples of the present invention are described in detail as above. For those of ordinary skill in the art, it is clear that these detailed descriptions are only preferred embodiments, and the scope of the present invention is not limited thereby. Accordingly, the substantial scope of the present invention will be defined by the appended claims and their equivalents.

**Claims**

1.   An array for detecting a target particle, comprising:

   a substrate with uneven surface,
   a lipid bilayer coating the uneven surface, and
   plural capturing substances binding to the target particle, wherein the capturing substances are localized in the lipid bilayer with fluidity, and labeled with a signaling material.

2.   The array for detecting a target particle of claim 1, wherein the uneven surface comprises a plurality of pillars, grooves, or a combination thereof.

3.   The array for detecting a target particle of claim 2, wherein diameter, height, or interval of the pillar or groove is at least 1.2 times the average diameter of the target particle.

4.   The array for detecting a target particle of claim 1, wherein the substrate is a solid substrate having hydrophilic surface or modified to have hydrophilic surface.

5.   The array for detecting a target particle of any one of claims 1 to 4, wherein the target particle is at least one selected from the group consisting of viruses, cells, proteins, and nucleic acid molecules.

6.   The array for detecting a target particle of claim 5, wherein the target particle is a virus.

7.   The array for detecting a target particle of claim 6, wherein the target particle is a coronavirus, an influenza virus, or a combination thereof.

8.   A kit for detecting a target particle, comprising two or more of the arrays for detecting a target particle of any one of claims 1 to 4.

9. The kit for detecting a target particle of claim 8, wherein the arrays are for detecting the same target particle.

10. The kit for detecting a target particle of claim 8, wherein the arrays are for detecting different target particles from each other.

11. The kit for detecting a target particle of claim 8, wherein the target particle is a virus.

12. The kit for detecting a target particle of claim 11, wherein the target particle is a coronavirus, an influenza virus, or a combination thereof.

13. A method of detecting a target particle, comprising:

contacting a sample with the array for detecting target particle of any one of claims 1 to 4 or a kit for detecting target particles comprising a plurality of the arrays; and
measuring a signal generated from the array or the kit.

14. The method of claim 8, wherein the sample is at least one selected from the group consisting of cells, blood, lymph, saliva, sputum, snivel, urine, and feces, obtained from a mammal.

15. The method of claim 13, wherein the target particle is a virus

16. The method of claim 15, wherein the target particle is a coronavirus, an influenza virus, or a combination thereof.

17. A kit for diagnosing virus infection, comprising a plurality of the arrays for detecting target particle of any one of claims 1 to 4, wherein the target particle is a virus.

18. The kit for diagnosing virus infection of claim 17, the virus is a coronavirus, an influenza virus, or a combination thereof.

【FIG. 1a】

【FIG. 1b】

[FIG. 2]

EP 4 141 446 A1

【FIG. 3a】

【FIG. 3b】

【FIG. 3c】

【FIG. 4】

【FIG. 5】

【FIG. 6】

【FIG. 7】

【FIG. 8】

【FIG. 9】

**INTERNATIONAL SEARCH REPORT**

| International application No. |
|---|
| **PCT/KR2021/004967** |

**A.    CLASSIFICATION OF SUBJECT MATTER**

**G01N 33/543**(2006.01)i; **G01N 33/569**(2006.01)i

According to International Patent Classification (IPC) or to both national classification and IPC

**B.    FIELDS SEARCHED**

Minimum documentation searched (classification system followed by classification symbols)

G01N 33/543(2006.01); B01L 3/00(2006.01); G01N 21/00(2006.01); G01N 33/50(2006.01); G01N 33/53(2006.01); G01N 33/569(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 지질 나노필러 어레이-기반 면역흡착분석(lipid nanopillar array-based immunosorbent assay), 지지형 지질 이중층(supported lipid bilayer), 필러(pillar), 홈(groove), 바이러스(virus)

**C.    DOCUMENTS CONSIDERED TO BE RELEVANT**

| Category* | Citation of document, with indication, where appropriate, of the relevant passages | Relevant to claim No. |
|---|---|---|
| X | LOU, Hsin-Ya et al. Functional lipid coating for the nanopillar-based capture of circulating tumor cells with high purity and efficiency. Langmuir. 06 January 2017, vol. 33, pp. 1097-1104. | 1-5,8-10,13,14 |
| | See abstract; page 1098, left column, third paragraph-right column; page 1100, left column, third paragraph; and figure 1. | |
| Y | | 6,7,11,12,15-18 |
| Y | HARTMAN, Kevin L. et al. Supported lipid bilayers as dynamic platforms for tethered particles. Nanoscale. 2015, vol. 7, pp. 66-76. | 6,7,11,12,15-18 |
| | See abstract; and figure 1. | |
| A | US 2019-0107539 A1 (INTERNATIONAL BUSINESS MACHINES CORPORATION) 11 April 2019 (2019-04-11) See entire document. | 1-18 |
| A | US 7514267 B1 (LOPEZ, Gabriel P. et al.) 07 April 2009 (2009-04-07) See entire document. | 1-18 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **03 August 2021** | **04 August 2021** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2021/004967**

**C.     DOCUMENTS CONSIDERED TO BE RELEVANT**

| Category* | Citation of document, with indication, where appropriate, of the relevant passages | Relevant to claim No. |
|---|---|---|
| A | KR 10-2015-0082729 A (SEOUL NATIONAL UNIVERSITY R&DB FOUNDATION) 16 July 2015 (2015-07-16)<br>    See entire document. | 1-18 |
| PX | KIM, Jieun et al. A Lipid-Nanopillar-Array-Based Immunosorbent Assay. Advanced Materials. (electronic publication) 25 May 2020, vol. 32, no. 26, 2001360 (pp. 1-6).<br>    See entire document. | 1-18 |

Form PCT/ISA/210 (second sheet) (July 2019)

**INTERNATIONAL SEARCH REPORT**
Information on patent family members

International application No.

**PCT/KR2021/004967**

| Patent document<br>cited in search report | | | Publication date<br>(day/month/year) | Patent family member(s) | | | Publication date<br>(day/month/year) |
|---|---|---|---|---|---|---|---|
| US | 2019-0107539 | A1 | 11 April 2019 | US | 10156568 | B2 | 18 December 2018 |
| | | | | US | 2016-320389 | A1 | 03 November 2016 |
| US | 7514267 | B1 | 07 April 2009 | US | 2009-0258372 | A1 | 15 October 2009 |
| | | | | US | 8334107 | B2 | 18 December 2012 |
| | | | | US | 8465983 | B1 | 18 June 2013 |
| KR | 10-2015-0082729 | A | 16 July 2015 | CN | 106461640 | A | 22 February 2017 |
| | | | | CN | 106461640 | B | 21 December 2018 |
| | | | | EP | 3093661 | A1 | 16 November 2016 |
| | | | | EP | 3093661 | B1 | 28 October 2020 |
| | | | | JP | 2017-503502 | A | 02 February 2017 |
| | | | | JP | 6402351 | B2 | 10 October 2018 |
| | | | | KR | 10-1568565 | B1 | 23 November 2015 |
| | | | | US | 10509030 | B2 | 17 December 2019 |
| | | | | US | 2017-0115286 | A1 | 27 April 2017 |
| | | | | WO | 2015-102475 | A1 | 09 July 2015 |

Form PCT/ISA/210 (patent family annex) (July 2019)

**EP 4 141 446 A1**